# EUROPÄISCHE PATENTSCHRIFT

(11) **EP 1 819 647 B1**
(45) Veröffentlichungstag und Bekanntmachung des Hinweises auf die Patenterteilung: **18.11.2009**
(21) Anmeldenummer: 05800877.2
(22) Anmeldetag: 27.09.2005
(51) Int. Cl.: C07B 35/02, C07C 45/62

(54) **VERFAHREN ZUR HYDRIERUNG VON , -UNGESÄTTIGTEN CARBONYLVERBINDUNGEN**
METHOD FOR HYDROGENATION OF , -UNSATURATED CARBONYL COMPOUNDS
PROCEDE D'HYDROGENATION DE COMPOSES CARBONYLE $G(A),$G(B)-INSATURES

(30) Priorität: 29.09.2004 DE 102004047794
(43) Veröffentlichungstag der Anmeldung: 22.08.2007
(73) Patentinhaber: Studiengesellschaft Kohle mbH, 45470 Mülheim an der Ruhr (DE)
(72) Erfinder: LIST, Benjamin, 45470 Mülheim an der Ruhr (DE); YANG, Jung, Woon, 45470 Mülheim an der Ruhr (DE)
(74) Vertreter: Christophersen, Ruth
(86) Internationale Anmeldenummer: PCT/DE2005/001705
(87) Internationale Veröffentlichungsnummer: WO 2006/034692

(56) Entgegenhaltungen:
- SÉVERINE TORCHY, GUY CORDONNIER, DIDIER BARBRY, JEAN JACQUES VANDEN EYNDE: MOLECULES, Bd. 7, 2002, Seiten 528-533, XP002358188
- YOSHIO INOUE, SHIN IMAIZUMI, HIROMITSU ITOH, TAKAO SHINYA: BULL CHEM SOC JPN, Bd. 61, 1988, Seiten 3020-3022, XP008057197
- KAORU NAKAMURA, MASAYUKI FUJTI, ATSUYOSHI OHNO, SHINZABURO OKA: TETRAHEDRON LETTERS, Bd. 25, 1984, Seiten 3983-3986, XP002358189

## Beschreibung

Die vorliegende Erfindung betrifft ein Verfahren zur Hydrierung von α,β-ungesättigten Carbonylverbindungen.

Verzweigte Carbonyle sind sehr wertvolle Intermediate in der Synthese von pharmazeutischen Verbindungen, Geruchsstoffen, Naturstoffen und anderen funktionellen Substanzen. So ist zum Beispiel das Citronellal ein in der β-Position verzweigter Aldehyd, der als Geruchsstoff und außerdem industriell in bedeutsamen Synthesen von Citronellol, Menthol, Muscone, und α-Tocopherol eingesetzt wird. Interessanterweise ist das aus natürlichen Quellen gewonnene Citronellal nicht enantiomerenrein. Ein Verfahren, dass seine enantiomerenreine Synthese erlaubt ist daher bedeutsam.

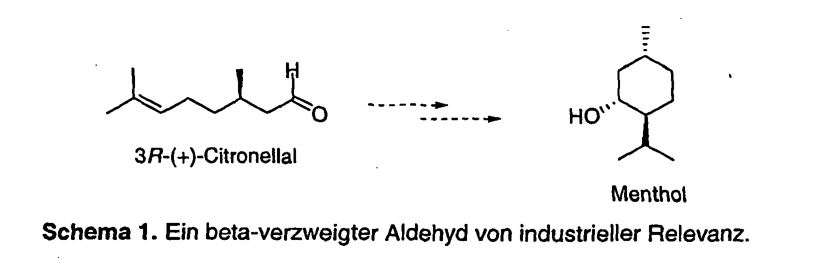

Mögliche Zugänge zu enantiomerenreinen verzweigten Carbonylverbindungen stellen katalytische asymmetrische Hydrierung bzw. conjugierte Reduktionen der entsprechenden α,β-ungesättigten Carbonylverbindungen dar.

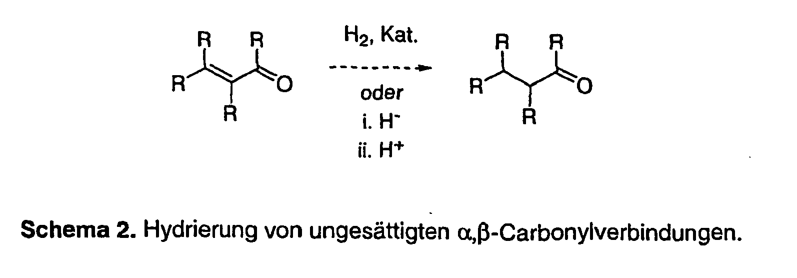

Eine Reihe von Beispielen für diese beiden Reaktionstypen sind in der Literatur beschrieben worden, alle basierend auf Metall-Katalyse, wobei katalytische asymmetrische Hydrierungen bisher nur erfolgreich mit Ketonen, jedoch nicht mit Aldehyden angewandt wurden. Hinzu kommt das Problem, dass existierende Verfahren oft nicht chemoselektiv sind. Oft werden hierbei neben der Doppelbindung auch weitere funktionelle Gruppen reduziert, was unerwünscht ist. Auch die Enantioselektivität ist oft unbefriedigend. Katalytisch asymmetrische Hydrierungen von Aldehyden sind bisher völlig unbekannt.

Ein weiterer Nachteil der Metall-Katalyse ist, dass als Hydrierungsagens H₂-Gas bei Drücken oberhalb von Atmosphärendruck verwendet wird. Der Einsatz von gasförmigen Reaktionspartnern erfordert regelmäßig einen hohen apparativen Aufwand. Hinzukommt, dass die Katalysatoren auf Edelmetallbasis sehr teuer sind. Die Katalysatoren müssen daher nicht nur aus dem Endprodukt abgetrennt sondern auch aufbereitet werden, damit die Edelmetall-Anteile in einem weiteren Verfahren einsetzen zu können.

Neben der Hydrierung mit Wasserstoff ist auch die Transfer-Hydrierung mit Wasserstoff-Donoren, wie 1,4-Dyhydropyridinen (Hantz'scher Ester), bekannt. So beschreiben Torchy et al. in Molecules 2002, 7, 528-533 die Hydrierung von Nitrostyrol mit unter Einsatz von Mikrowellen. Inoue et al. haben zur selektiven Reduktion von Doppelbindungen α,β-ungesättigte Carbonylverbindungen mit Hantz'schen Estern in Gegenwart von Essigsäure umgesetzt (Bull. Chem. Soc. Jpn., 61, 3020-3022 (1988*)*. Nakamura et al. offenbaren in Tetrahedron Letter, Vol. 25, No. 36, 3983-3986, 1984, die Umsetzung von α,β-ungesättigte Carbonylverbindungen mit Hantz'schen Estern auf Silicagel. Die im Stand der Technik beschriebenen Verfahren haben den Nachteil, dass sie nur mit Ketonen nicht aber mit Aldehyden durchgeführt werden können.

Der vorliegenden Erfindung lag die Aufgabe zugrunde ein einfaches Verfahren zur Herstellung von verzweigten Carbonylverbindungen zur Verfügung zu stellen, bei welchem auf die Metallkatalysatoren verzichtet werden kann und wobei das Verfahren nicht auf die Ketone beschränkt sondern auch auf Aldehyde anwendbar sein soll.

Gegenstand der vorliegenden Erfindung ist Verfahren zur Hydrierung von α,β-ungesättigten Carbonylverbindungen, in welchem eine Verbindung mit der allgemeinen Formel I 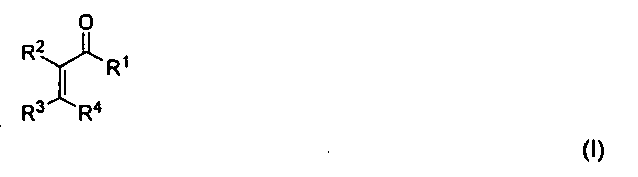 in der
R¹ für H, einen verzweigten oder unverzweigten, gesättigten oder ungesättigten Kohlenwasserstoffrest mit 1 bis 30 Kohlenstoffatomen, der geeignete Substituenten haben kann, wobei der Kohlenwasserstoffrest ein oder mehrere Heteroatome in der Kette aufweisen kann, eine Arylgruppe oder Heteroarylgruppe, die geeignete Substituenten haben können, steht,
R², R³ und R⁴ gleich oder verschieden sein können und für H, F, Cl, Br, J, OH, CN, NO₂, NO, SO₂, SO₃⁻, Amino, Mono- und Di-(C₁-C₂₄-alkyl)-substituiertes Amino, Mono- und Di-(C₅-C₂₀-aryl)-substituiertes Amino, Imino, Phosphono, Phosphonato, Phosphinato, Phospho, Phosphino, einen verzweigten oder unverzweigten, gesättigten oder ungesättigten Kohlenwasserstoffrest mit 1 bis 30 Kohlenstoffatomen, der geeignete Substituenten haben kann, wobei der Kohlenwasserstoffrest ein Heteroatom in der Kette aufweisen kann, eine Arylgruppe oder Heteroarylgruppe, die geeignete Substituenten haben können, stehen,
wobei jeweils 2 oder mehr der Reste R¹, R², R³ und R⁴ einen 5- oder 6-gliedrigen Ring oder annellierte 5-gliedrige und/oder 6-gliedrige Ringe bilden können, die aromatisch, alicyclisch, heteroaromatisch oder heteroalicyclisch sein können und bis zu 4 Substituenten aufweisen können,
mit einem Hydrid-Donor unter Bildung einer Verbindung mit der allgemeinen Formel II 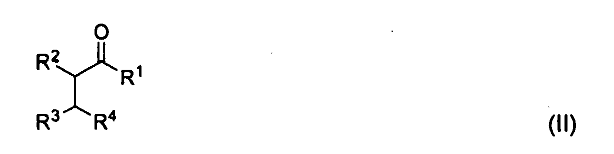
in der R¹, R², R³ und R⁴ wie oben definiert sind,
umgesetzt wird, das dadurch gekennzeichnet ist, dass die Umsetzung in Gegenwart eines Katalysators durchgeführt wird, der ausgewählt ist aus organischen Basen, insbesondere Aminen und deren Säureadditionssalzen.

Durch das erfindungsgemäße Verfahren können hoch chemoselektiv α,β-ungesättigte Carbonylverbindungen katalytisch reduziert werden, wobei auf den Einsatz von Metallkatalysatoren verzichtet werden kann. Es wurden zwei Varianten des Verfahrens entwickelt, welche sich einerseits für nicht-asymmetrische Reduktionen unter Bildung von racemischen Gemischen und andererseits für hoch enantioselektive Reduktionen eignen.

Ein Beispiel für den Verfahrensablauf ist im nachfolgenden Schema 3 am Beispiel der Umsetzung einer α,β-ungesättigten Carbonylverbindung mit einem Dihydropyridin als Hydrid-Donor dargestellt. In diesem Schema wird ein Amin oder ein Ammoniumsalz als Katalysator eingesetzt.

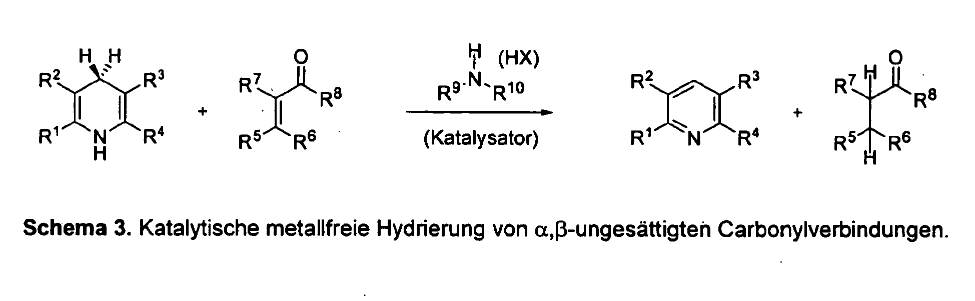

Die Reste R¹, R², R³ und R³ können die oben genannte Bedeutung haben. Der verzweigte oder unverzweigte, gesättigte oder ungesättigte Kohlenwasserstoffrest mit 1 bis 30 Kohlenstoffatomen ist vorzugsweise ausgewählt aus C₁-C₂₄-Alkyl, C₂-C₂₄-Alkenyl, C₂-C₂₄-Alkinyl, C₁-C₂₄-Alkoxy, C₂-C₂₄-Alkenyloxy, C₂-C₂₄-Alkinyloxy, C₅-C₃₀-Aryl, C₅-C₃₀-Aryloxy, C₂-C₂₄-Alkoxyalkyl, C₆-C₃₀-Aryloxyalkyl, Hydroxyl, Sulfhydryl, C₂-C₂₄-Alkylcarbonyl, C₆-C₃₀-Arylcarbonyl, C₂-C₂₄-Alkoxycarbonyl, C₆-C₃₀-Aryloxycarbonyl, Halogencarbonyl, C₂-C₂₄-Alkylcarbonato, C₆-C₃₀-Arylcarbonato, Carboxy, Carboxylato, Carbamoyl, Mono- und Di-(C₁-C₂₄-alkyl)-substituiertes Carbamoyl, C₂-C₂₄-Alkylamido, C₆-C₃₀-Arylamido, C₂-C₂₄-Alkylimino. C₆-C₃₀-Arylimino, C₁-C₂₄-Alkylsulfanyl, C₅-C₃₀-Arylsulfanyl, C₁-C₂₄-Alkylsutfinyl, C₅-C₃₀-Arylsulfinyl, C₁-C₂₄-Alkylsulfonyl, C₅-C₃₀-Aryisuffonyl.

Der verwendete Begriff "Alkyl" bedeutet einen linearen, verzweigten oder cyclischen Kohlenwasserstoffrest, der üblicherweise 1 bis 30, vorzugsweise 1 bis 24 Kohlenstoffatome aufweist, wie Methyl, Ethyl, n-Propyl, Isopropyl, n-Butyl, Isobutyl, t-Butyl, Octyl, Decyl usw., aber auch Cycloalkylgruppen wie Cyclopentyl, Cyclohexyl usw. Vorzugsweise weisen die Kohlenwasserstoffreste 1 bis 18, insbesondere 1 bis 12 Kohlenstoffatome auf.

Der verwendete Begriff "Alkenyl" bedeutet einen linearen, verzweigten oder cyclischen Kohlenwasserstoffrest mit mindestens einer Doppelbindung, der üblicherweise 2 bis 30, vorzugsweise 2 bis 24 Kohlenstoffatome ausweist, wie Ethenyl, n-Propenyl, Isopropenyl, n-Butenyl, Isobutenyl, Octenyl, Decenyl, Tetradecenyl, Hexadecenyl, Eicosenyl, Tetracosenyl usw., aber auch Cycloalkenylgruppen wie Cyclopentenyl, Cyclohexenyl usw. Vorzugsweise weisen die Alkenylreste 2 bis 18, insbesondere 2 bis 12 Kohlenstoffatome auf.

Der verwendete Begriff "Alkinyl" bedeutet einen linearen, verzweigten oder cyclischen Kohlenwasserstoffrest mit mindestens einer Dreifachbindung, der üblicherweise 2 bis 30, vorzugsweise 2 bis 24 Kohlenstoffatome aufweist, wie Ethinyl, n-Propinyl, Isopropinyl, n-Butinyl, Isobutinyl, Octinyl, Decinyl, Tetradecinyl, Hexadecinyl, Eicosinyl, Tetracosinyl usw., Vorzugsweise weisen die Alkînylreste 2 bis 18, insbesondere 2 bis 12 Kohlenstoffatome auf.

Bevorzugte Alkoxygruppen sind Methoxy, Ethoxy, n-Propoxy, Isopropoxy, n-Butyloxy, Isobutyoxy, t-Butyloxy, Pentyloxy, Hexyloxy usw.

Als Arylgruppen werden im Rahmen der vorliegenden Erfindung aromatische Ringsysteme mit 5 bis 30 Kohlenstoffatomen und ggf. Heteroatomen wie N, O, S, P, Si, im Ring verwendet, wobei die Ringe einfache oder mehrfache Ringsysteme, z. B. kondensierte Ringsysteme oder über einfache Bindungen oder Mehrfachbindungen aneinander gebundene Ringe sein können. Beispiele für aromatische Ringe sind Phenyl, Naphthyl, Biphenyl, Diphenylether, Diphenylamin, Benzophenon und dergleichen. Substituierte Arylgruppen weisen einen oder mehrere Substituenten auf, wie sie bereits in der Definition von R¹ angegeben wurden. Beispiele für Heteroalkylgruppen sind Alkoxyaryl, Alkylsulfanylsubstituiertes Alkyl, N-alkyliertes Aminoalkyl und dergleichen. Beispiele für Heteroarylsubstituenten sind Pyrrolyl, Pyrrolidinyl, Pyridinyl, Chinolinyl, Indolyl, Pyrimidinyl, Imidazolyl, 1,2,4-Triazolyl, Tetrazolyl, und dergleichen. Als Beispiele für Heteroatom-enthaltende Alicyclische Gruppen können Pyrrolidino, Morpholino, Piperazino, Piperidino unsw. genannt werden.

Als Substituenten, die die voranstehend genannten Gruppen aufweisen können, können OH, F, Cl, Br, J, CN, NO₂, NO, SO₂, SO₃⁻, Amino, Mono- und Di-(C₁-C₂₄-alkyl)-substituiertes Amino, Mono- und Di-(C₅-C₂₀-aryl)-substituiertes Amino, Imino, die wiederum substituiert sein können. Insbesondere die cyclischen Reste können auch C₁-C₆-Alkylgruppen als Sustituenten aufweisen.

Als funktionelle Gruppen, können die voranstehend definierten Reste oder die Substituenten alle dem Fachmann bekannten und in der organischen Synthese üblichen Gruppen enthalten, wie Halogen, Hydroxyl, Sulfhydryl, C₁-C₂₄-Alkoxy, C₂-C₂₄-Alkenyloxy, C₂-C₂₄-Alkynyloxy, C₅-C₂₀-Aryloxy, Acyl sowie C₂-C₂₄-Alkylcarbonyl (-CO-Alkyl) und C₆-C₂₀-Arylcarbonyl (-CO-aryl), Acyloxy (-O-acyl), C₂-C₂₄-Alkoxycarbonyl (-(CO)-O-Alkyl), C₆-C₂₀-Aryloxycarbonyl (-(CO)-O-aryl, Halogencarbonyl (-CO)-X, worin X is Halogen bedeutet), C₂-C₂₄-Alkylcarbonato (-O-(CO)-O-Alkyl), C₆-C₂₀-Arylcarbonato (-O-(CO)-O-aryl), Carboxy (-COOH), Carboxylato (-COO⁻), Carbamoyl (-(CO)-NH₂), mono-substituiertes C₁-C₂₄-Alkylcarbamoyl (-(CO)-NH(C₁-C₂₄ alkyl)), disubstituiertes Alkylcarbamoyl (-(CO)-N(C₁-C₂₄-alkyl)₂), monosubstituiertes Arylcarbamoyl (-(CO)-NH-aryl), Thiocarbamoyl (-(CS)-NH₂), Carbamido (-NH-(CO)-NH₂), Cyano (-C≡N), Isocyano -N⁺≡C⁻), Cyanato (-O-C≡N), isocyanato (-O-N⁺≡C⁻), Isothiocyanato (-S-C≡N), Azido (-N=N⁺=N⁻), Formyl (-(CO)-H), Thioformyl (-(CS)-H), Amino (-NH₂), mono- and di-(C₁-C₂₄-alkyl)-substituiertes Amino, mono-and di-(C₅-C₂₀aryl)-substituiertes Amino, C₂-C₂₄-Alkylamido (-NH-(CO)-alkyl), C₅-C₂₀-Arylamido (-NH-(CO)-aryl), Imino (-CR=NH, worin R = H, C₁-C₂₄-Alkyl, C₅-C₂₀-Ary), C₆-C₂₀-Alkaryl, C₆-C₂₀-Aralkyl, etc.), Alkylimino (-CR=N(alkyl), worin R = H, Alkyl, Aryl, Alkaryl, etc.), Arylimino (-CR=N(aryl), worin R = H, Alkyl, Aryl, Alkaryl, etc.), Nitro (-NO₂), Nitroso (-NO), Sulfo (-SO₂-OH), Sulfonato (-SO₂-O⁻), C₁-C₂₄-Alkylsulfanyl (-S-alkyl; auch als "Alkylthio" bezeichnet), Arylsulfanyl (-S-aryl; auch als "arylthio" bezeichnet), C₁-C₂₄-Aalkylsuifinyl (-(SO)-alkyl), C₅-C₂₀-Arylsulfinyl (-(SO)-aryl), C₁-C₂₄-Alkylsulfonyl (-SO₂-alkyl), C₅-C₂₀-Arylsulfonyl (-SO₂-aryl), Phosphono (-P(O)(OH)₂), Phosphonato (-P(O)(O⁻)₂), Phosphinato (-P(O)(O⁻)), Phospho (-PO₂), und Phosphino (-PH₂); und die Kohlenwasserstoffreste C₁-C₂₄-Alkyl, vorzugsweise C₁-C₁₈-Alkyl, besonders bevorzugt C₁-C₁₂-Alkyl, insbesondere C₁-C₆-Alkyl, C₂-C₂₄-Alkenyl, vorzugsweise C₂-C₁₈-Alkenyl, besonders bevorzugt C₂-C₁₂-Alkenyl, insbesondere C₂-C₆-Alkenyl, C₂-C₂₄-Alkynyl, vorzugsweise C₂-C₁₈-Alkynyl, besonders bevorzugt C₂-C₁₂-Alkynyl, insbesondere C₂-C₆-Alkynyl, C₅-C₃₀-Aryl, vorzugsweise C₅-C₂₀-Aryl, besonders bevorzugt C₅-C₁₂-Aryl und C₆-C₃₀-Aralkyl, vorzugsweise C₆-C₂₀-Aralkyl, besonders bevorzugt C₆-C₁₂-Aralkyl.

Die Verbindungen mit der Formel I werden erfindungsgemäß mit einem Hydriddonor umgesetzt. Als Hydrid-Donoren kommen alle Verbindungen in Betracht die Hydrid-lonen freisetzen können, wobei organische Hydrid-Donoren bevorzugt sind. Geeignete Hydrid-Donoren sind z. B. Hantzsch Dihydropyridin. In einer bevorzugten Ausführungsform der vorliegenden Erfindung ist wird ein Dihydropyridin der allgemeinen Formel III eingesetzt. 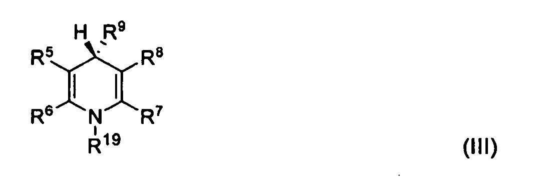 in der R⁵, R⁶, R⁷, R⁸ und R⁹ gleich oder verschieden sein können und für H, OH, einen gesättigten oder ungesättigten, geradkettigen, verzweigten oder cyclischen unsubstituierten oder substituierten C₁-C₂₀-Alkylrest, Halogen, insbesondere F, Cl, Br, J, NO₂, eine Aminogruppe, -CO₂R¹⁰, C(O)R¹¹, C-O-R¹², OR¹³, wobei R¹⁰ bis R¹³ ausgewählt sein können aus H, verzweigten oder unverzweigten, gesättigten oder ungesättigten Kohlenwasserstoffresten mit 1 bis 30 Kohlenstoffatomen, die geeignete Substituenten haben können, wobei die Kohlenwasserstoffreste ein oder mehrere Heteroatome in der Kette aufweisen können, Arylgruppen oder Heteroarylgruppen, die ihrerseits geeignete Substituenten haben können, stehen, und
R¹⁹ für H, einen verzweigten oder unverzweigten, gesättigten oder ungesättigten Kohlenwasserstoffrest mit 1 bis 30 Kohlenstoffatomen, der geeignete Substituenten enthalten kann, wobei die Kohlenwasserstoffreste ein oder mehrere Heteroatome in der Kette sowie geeignete Substituenten aufweisen können, Arylgruppen oder Heteroarylgruppen, die ihrerseits geeignete Substituenten haben können, steht,
sowie Salzen der Verbindungen mit der Formel III.

In den Verbindungen mit der Formel III sind R⁵ und R⁸ vorzugsweise ausgewählt aus elektronenziehenden Reste, wie Halogen, NO₂, -CO₂R¹⁰, C(O)R¹¹, C-O-R¹², OR¹³, wobei R¹⁰ bis R¹³ die oben angegebene Bedeutung haben können. R⁶ und R⁷ sind vorzugsweise ausgewählt aus H oder einer C₁-C₆-Alkylgruppe, R⁹ ist vorzugsweise Wasserstoff oder eine C₁-C₆-Alkylgruppe und R¹⁹ steht vorzugsweise für H oder eine C₁-C₆-Alkylgruppe.

Als Beispiele für weitere Hydrid-Donatoren können sekundäre Alkohole, Silane, Triarylmethane, Cyclohexadiene, Formaldehyd und seine Derivate, Ameisensäure und deren Derivate und Salze genannt werden.

Die Selektivität und Effizienz des erfindungsgemäßen Verfahrens können weiter verbessert werden, wenn die Reaktion in Gegenwart eines Katalysators durchgeführt wird. Die Durchführung der Reaktion in Gegenwart eines Katalysators kann in unterschiedlichen Ausgestaltungen erfolgen. In einer möglichen Ausführungsform ist der Katalysator ausgewählt aus organischen Basen, insbesondere primären und sekundären Aminen und deren Säureadditionssalzen, insbesondere Aminen mit der allgemeine Formel IV

NHR¹⁴R¹⁵ (IV)

in der
R¹⁴ für H, eine Kohlenwasserstoffgruppe, wie eine gesättigte oder ungesättigte, verzweigte oder lineare Alkylgruppe, Alkenylgruppe, Alkinylgruppe, Aryl, Alkylaryl, die geeignete Substituenten haben kann, eine Heteroatom-enthaltende Kohlenwasserstoffgruppe, die geeignete Substituenten haben kann, und
R¹⁵ für eine Kohlenwasserstoffgruppe, wie eine gesättigte oder ungesättigte, verzweigte oder lineare Alkylgruppe, Alkenylgruppe, Alkinylgruppe, Aryl, Alkylaryl, die geeignete Substituenten haben kann, eine Heteroatom-enthaltende Kohlenwasserstoffgruppe, die geeignete Substituenten haben kann, steht,
wobei R¹⁴ und R¹⁵ einen anellierten, substituierten oder unsubstituierten Ring mit 3 bis 7 Kohlenstoffatomen bilden können, der zusätzlich zum N-Atom aus Formel IV ggf. eine weiteres Heteroatom enthalten kann,
und deren Säureadditionssalze.

Wenn R¹⁴ und R¹⁵ gemeinsam einen Ring bilden, werden die Reste so ausgewählt, dass vorzugsweise ein 5- oder 6 gliedriger alicyclischer oder aromatischer Ring erhalten wird, wie Pyrrolidiny, Piperidinyl, Morpholinyl, Pyrrolyl, Pyridinyl, Pyrimidinyl, Imidazolyl etc.

Zur Bildung der Säureadditionssalze geeignete Säure sind ausgewählt aus anorganischen Säuren, insbesondere aus HCl, H₂SO₄, H₂SO₃, HNO₃, HNO₂, HClO₄, H₃PO₄, Chromsäure und geeigneten Kombinationen daraus, und organischen Säuren, insbesondere Carbonsäuren, Sulfonsäuren, Phosphonsäuren, Phenolen mit 1 bis 5 elektronenziehenden Substituenten. Beispiele für geeignete organische Säuren sind Essigsäure, Propionsäure, Glykolsäure, Brenztraubensäure, Oxalsäure, Äpfelsäure, Malonsäure, Bernsteinsäure, Maleinsäure, Fumarsäure, Weinsäure, Citronenensäure, Benzoesäure, 2-Nitrobenzoesäure, Zimtsäure, Mandelsäure, Methansulfonsäure, Ethansulfonsäure, Triflourmethansäure, p-Toluolsulfonsäure, Salicylsäure, Chloressigsäure, Dichloressigsäure, Trichloressigsäure, Trifluoressigsäure und Kombinationen daraus.

Bevorzugt eingesetzte Verbindungen mit der Formel III sind sekundäre Amine, d.h. R¹⁴ steht nicht für Wasserstoff. Für die nicht-asymmetrischen Verfahrensweise werden vorzugsweise nicht-chirale Amine eingesetzt, z. B. Verbindungen, in denen R¹⁴ und R¹⁵ jeweils unabhängig voneinander ausgewählt sind aus Methyl, Ethyl, Propyl, Butyl, Cyclopentyl, Cyclohexyl, Cyclooctyl, Phenyl, Naphthyl, Benzyl oder Trimethylsilyl. Weiter bevorzugte Amine sind solche, in denen das N-Atom und die Reste R¹⁴ und R¹⁵ einen 3 bis 15-gliedrigen, ggf. substiutierten Ring bilden. Besonders bevorzugt werden Amine mit mindestens 1 chiralen Zentrum im Molekül eingesetzt. Als besonders geeignet haben sich Imidazolidinon und dessen Derivate mit der allgemeinen Formel V erwiesen: 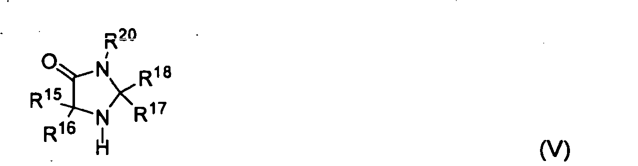 in der
R¹⁵_{,} R¹⁶, R¹⁷ und R¹⁸ gleich oder verschieden sein können und für H, OH, F, Cl, Br, J, NO₂, NO, SO₂, SO₃⁻, Amino, Mono- und Di-(C₁-C₂₄-alkyl)-substituiertes Amino, Mono- und Di-(C₅-C₂₀-aryl)-substituiertes Amino, Imino, Phosphono, Phosphonato, Phosphinato, Phospho, Phosphino, einen verzweigten oder unverzweigten, gesättigten oder ungesättigten Kohlenwasserstoffrest mit 1 bis 30 Kohlenstoffatomen, der geeignete Substituenten haben kann, wobei der Kohlenwasserstoffrest ein oder mehrere Heteroatome in der Kette aufweisen kann, eine Arylgruppe oder Heteroarylgruppe, die geeignete Substituenten haben können, stehen, wobei die Reste R¹⁷ und/oder R¹⁸ mit R²⁰ einen 5- oder 6-gliedrigen Ring bilden können, der aromatisch, alicyclisch, heteroaromatisch oder heteroalicyclisch sein kann und bis zu 4 Substituenten aufweisen kann,
R²⁰ für H, eine Kohlenwasserstoffgruppe, wie eine gesättigte oder ungesättigte, verzweigte oder lineare Alkylgruppe, Alkenylgruppe, Alkinylgruppe, Aryl, Alkylaryl, die geeignete Substituenten haben kann, eine Heteroatom-enthaltende Kohlenwasserstoffgruppe, die geeignete Substituenten haben kann, steht.

Der Katalysator wird vorzugsweise in einer Menge von 0,1 bis 100 Mol-%, insbesondere von 1 bis 30 Mol-%, und besonders bevorzugt in einer Menge von 0,1 bis 10 Mol-%, bezogen auf die Menge der Verbindung mit der Formel I, eingesetzt.

In einer weiteren Ausgestaltung der vorliegenden Erfindung kann das als Hydrid-Donor genannte Dihydropyridin mit der allgemeinen Formel III, neben dem eigentlichen Katalysator der Formel IV auch als Co-Katalysator eingesetzt werden. In dieser Ausführungsform wird zum Reaktionsgemisch H₂ zugeführt, das das nach Abgabe von Hydrid zum Pyridin oxidierte Dihydropyridin unmittelbar wieder reduziert, welches dann erneut zur Hydrierung von Carbonylverbindung zur Verfügung steht. Diese Verfahrensweise hat den Vorteil, dass die Reaktionskomponte des Hydrid-Donors nur in geringen Mengen eingesetzt werden kann und nur das relativ preiswerte H₂-Gas kontinuierlich zugeführt werden muss. Die Reduktion des Pyridin durch H₂ kann z. B. durch die Gegenwart eines geeigneten chemischen Katalysators oder eines Enzyms katalysiert werden. Alternativ zu H₂ können auch andere Reduktionsmittel oder elektrochemische Methoden eingesetzt werden.

Zur Durchführung des erfindungsgemäßen Verfahrens werden die Ausgangssubstanzen, d.h. die Verbindungen mit der Formel I, der Hydrid-Donor und der ggf. eingesetzte Katalysator in einem geeigneten Lösungsmittel, dass die Reaktion nicht negativ beeinflusst, gelöst bzw. suspendiert. Liegt eine der Reaktionskomponenten als Gas vor (beispielsweise der Hydrid-Donor), werden die festen bzw. flüssigen Komponenten in einem Lösungsmittel gelöst oder suspendiert vorgelegt und die gasförmige Komponente anschließend eingeleitet.

Die Reaktion wird vorzugsweise bei Normaldruck durchgeführt. Liegt einer Reaktionspartner als Gas vor, z. B. der Hydrid-Donor, kann die Reaktion auch bei höheren Drücken, insbesondere von 0,1 bis 200 bar, bevorzugt 0,5 bis 50 und besonders bevorzugt 0,5 bis 5 bar, durchgeführt werden.

Die Reaktionstemperatur ist unproblematisch, die Reaktion kann über einen Bereich zwischen -100°C und 100°C, vorzugsweise zwischen -90°C und 50°C, durchgeführt werden. Um die Bildung von Nebenprodukten möglichst zu vermeiden wird die Reaktion vorzugsweise in Inertgasatmosphäre durchgeführt.

Nach Abschluß der Reaktion kann das erhaltene Reaktionsprodukt in an sich bekannter Weise isoliert werden. Üblicherweise wird das Lösungsmittel entfernt und das erhaltene Rohprodukt durch dem Fachmann gut bekannte Verfahren, wie Chromatographie, Destillation, Sublimation, Kristallisation, Umkristallisation, Extraktion usw. gereinigt werden.

### Beispiele

### 1. Nicht-asymmetrische konjugierte Reduktion von Enal 3a und 3l durch Hantzsch Ester 1 katalysiert durch das Dibenzylammonium Salz der Trifluoressigsäure.

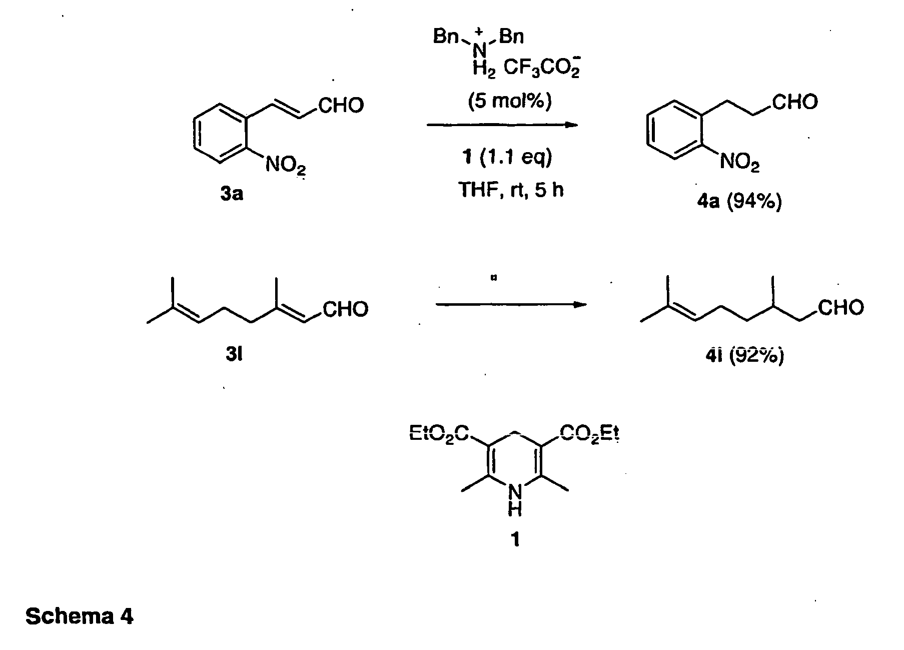

### Beispiel 1: Synthese des Aldehyds (o-Nitrophenyl)-propanal (4a):

Zu einer Lösung von o-Nitrozimtaldehyd (**3a**, 88.6 mg, 0.5 mmol) and Katalysator **2a** (7.8 mg, 0.025 mmol, 5 mol%) in wasserfreiem THF (2 mL) wurde das Dihydropyridin **1** (140 mg, 0.55 mmol, 1.1 eq) zugefügt. Das Reaktionsgemisch wurde bei Raumtemperatur 5 h unter Argon gerührt. Anschließend wurde das Lösungsmittel entfernt und der Rückstand wurde über Silicagel (30 % Diethylether/n-hexan) chromatographiert. Es wurden 84 mg (94%) 3-(o-Nitrophenyl)propanal (**4a**) als Öl erhalten.

Analog Beispiel 1 wurden die in nachfolgender Tabelle dargestellten Verbindungen umgesetzt bzw. erhalten.

| | | | |
|---|---|---|---|
| 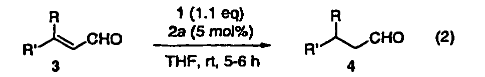 | | | |

| Entry | Substrate | Product | Yield (%)^{a} |
|---|---|---|---|
| (1) | 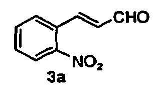 | 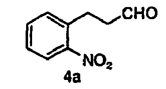 | 94 |
| (2) | 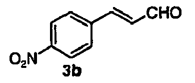 | 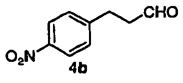 | 96 |
| (3) | 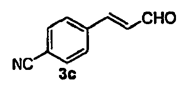 | 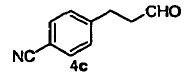 | 93 |
| (4) | 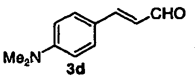 | 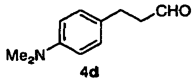 | 81^{b} |
| (5) | 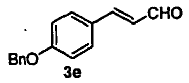 | 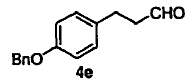 | 92 |
| (6) | 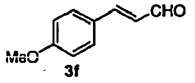 | 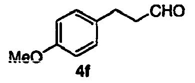 | 92 |
| (7) | 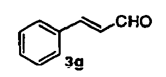 | 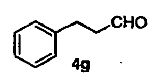 | 92° |
| (8) | 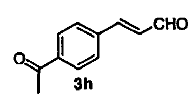 | 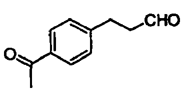 | 90 |
| (9) | 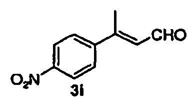 | 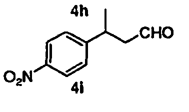 | 94 |
| (10) | 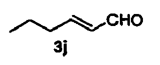 | 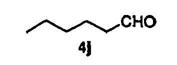 | 90^{b,c} |
| (11) | 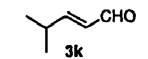 | 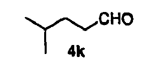 | 86^{b,c} |
| (12) | 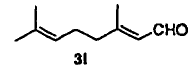 | 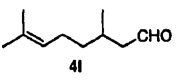 | 92 |

### 2. Asymmetrische konjugierte Reduktion von Enalen mit Katalysator 5.

### Beispiel 2: Synthese von (R)-4-(1-Methyl-3-oxo-propyl)-benzonitril :

Zu einer Lösung von (*E*)- oder (*Z*)-4-(1-Methyl-3-oxo-propenyl)-benzonitril (0.5 mmol) (oder einer E/Z Mischung) und Katalysator **5** (10 mol%) in wasserfreiem Dioxane (7 mL) wurde das Dihydropyridin **6** (1.1 eq) zugefügt. Das Reaktionsgemisch wurde bei 13°C für 36 h unter Argon gerührt. Anschließend wurde das Lösungsmittel entfernt und der Rückstand wurde über Silicagel chromatographiert. Als Produkt wurde (*R*)-4-(1-Methyl-3-oxo-propyl)-benzonitril in 90% Ausbeute und in einem Enantiomerenverhältnis von 97,5:2,5 erhalten.

Analog wurden Diverse andere ungesättigte Aldehyde umgesetzt (Schema 6).

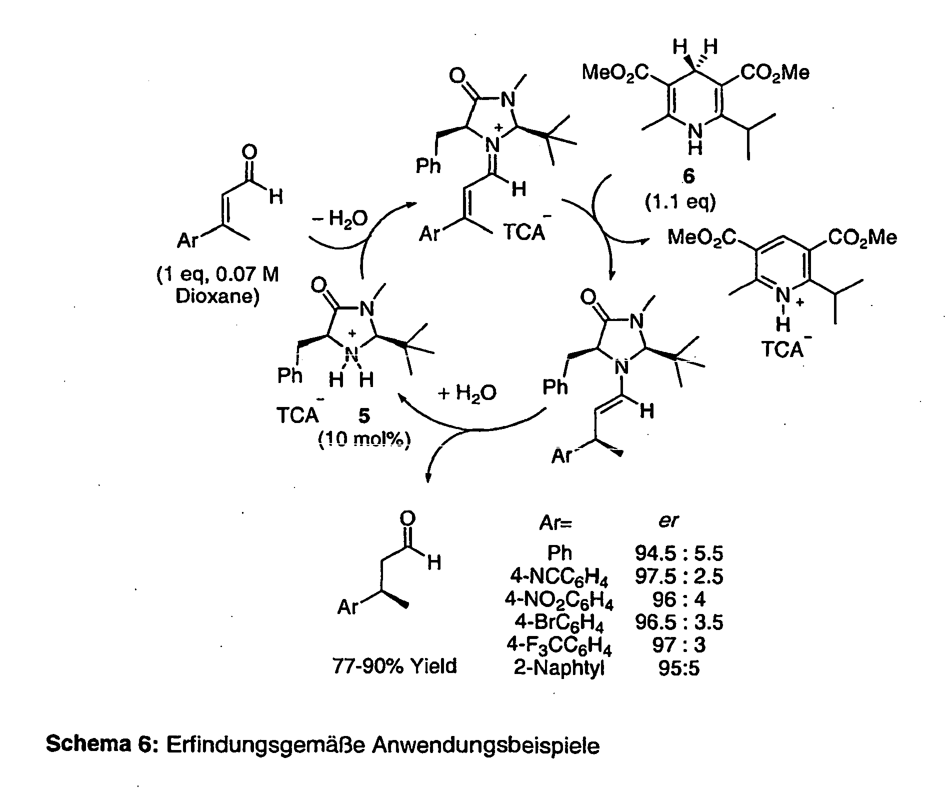

### Beispiel 3: Hydrierung eines Ketons

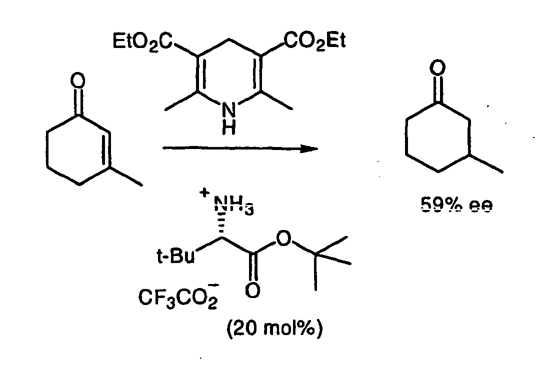

Zu einer Lösung von 3-Methyl-cyclohexenon (0.5 mmol) and Katalysator (0.1 mmol, 20 mol%) in wasserfreiem 1,4-Dioxan (2 mL) wurde das Dihydropyridin **1** (1.1 eq) zugefügt. Das Reaktionsgemisch wurde bei Raumtemperatur **4** Tage unter Argon gerührt. Anschließend wurde das Lösungsmittel entfernt und der Rückstand wurde über Silicagel (30 % Diethylether/n-hexan) chromatographiert. Das Keton wurde mit einem ee-Wert von 59% isoliert.

## Patentansprüche

1. Verfahren zur Hydrierung von α,β-ungesättigten Carbonylverbindungen, in welchem eine Verbindung mit der allgemeinen Formel I 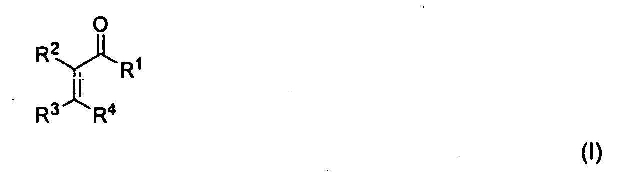 in der
R¹ für H, einen verzweigten oder unverzweigten, gesättigten oder ungesättigten Kohlenwasserstoffrest mit 1 bis 30 Kohlenstoffatomen, der geeignete Substituenten haben kann, wobei der Kohlenwasserstoffrest ein oder mehrere Heteroatome in der Kette aufweisen kann, eine Arylgruppe oder Heteroarylgruppe, die geeignete Substituenten haben können, steht,
R², R³ und R⁴ gleich oder verschieden sein können und für H, F, Cl, Br, J, OH, CN, NO₂, NO, SO₂, SO₃⁻, Amino, Mono- und Di-(C₁-C₂₄-alkyl)-substituiertes Amino, Mono-und Di-(C₅-C₂₀-aryl)-substituiertes Amino, Imino, Phosphono, Phosphonato, Phosphinato, Phospho, Phosphine, einen verzweigten oder unverzweigten, gesättigten oder ungesättigten Kohlenwasserstoffrest mit 1 bis 30 Kohlenstoffatomen, der geeignete Substituenten haben kann, wobei der Kohlenwasserstoffrest ein Heteroatom in der Kette aufweisen kann, eine Arylgruppe oder Heteroarylgruppe, die geeignete Substituenten haben können, stehen,
wobei jeweils 2 oder mehr der Reste R¹, R², R³ und R⁴ einen 5- oder 6-gliedrigen Ring oder annellierte 5-gliedrige und/oder 6-gliedrige Ringe bilden können, die aromatisch, alicyclisch, heteroaromatisch oder heteroalicyclisch sein können und bis zu 4 Substituenten aufweisen können,
mit einem Hydrid-Donor unter Bildung einer Verbindung mit der allgemeinen Formel II 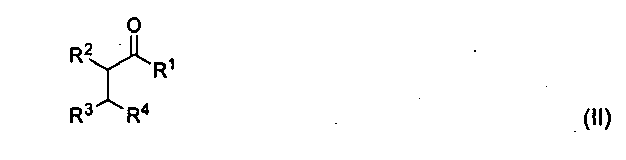
in der R¹, R², R³ und R⁴ wie oben definiert sind,
umgesetzt wird, **dadurch gekennzeichnet, dass** die Umsetzung in Gegenwart eines Katalysators durchgeführt wird, der ausgewählt ist aus organischen Basen, insbesondere Aminen und deren Säureadditionssalzen.

2. Verfahren nach Anspruch 1, **dadurch gekennzeichnet, dass** der Hydrid-Donor ausgewählt ist aus Dihydropyridinen, sekundären Alkoholen, Silanen, Triarylmethanen, Cyclohexadienen, Formaldehyd und seine Derivaten, Ameisensäure und deren Derivaten und Salzen.

3. Verfahren nach Anspruch 2, **dadurch gekennzeichnet, dass** als Hydrid-Donor ein Dihydropyridin der allgemeinen Formel III eingesetzt. 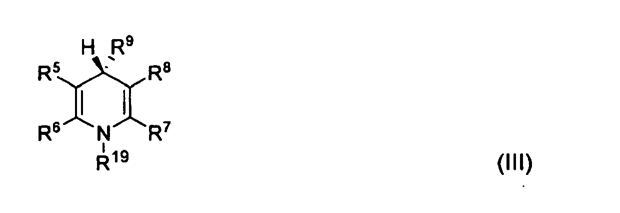
in der R⁵, R⁶, R⁷, R⁸ und R⁹ gleich oder verschieden sein können und für H, OH, einen gesättigten oder ungesättigten, geradkettigen, verzweigten oder cyclischen, unsubstituierten oder substituierten C₁-C₂₀-Alkylrest, Halogen, insbesondere F, Cl, Br, J, NO₂, eine Aminogruppe, -CO₂R¹⁰, C(O)R¹¹, C-O-R¹², OR¹³, wobei R¹⁰ bis R¹³
ausgewählt sein können aus H, verzweigten oder unverzweigten, gesättigten oder ungesättigten Kohlenwasserstoffresten mit 1 bis 30 Kohlenstoffatomen, die geeignete Substituenten haben können, wobei die Kohlenwasserstoffreste ein oder mehrere Heteroatome in der Kette aufweisen können, Arylgruppen oder Heteroarylgruppen, die ihrerseits geeignete Substituenten haben können, stehen und
R¹⁹ für H, einen verzweigten oder unverzweigten, gesättigten oder ungesättigten Kohlenwasserstoffrest mit 1 bis 30 Kohlenstoffatomen, der geeignete Substituenten enthalten kann, wobei die Kohlenwasserstoffreste ein oder mehrere Heteroatome in der Kette sowie geeignete Substituenten aufweisen können, Arylgruppen oder Heteroarylgruppen, die ihrerseits geeignete Substituenten haben können, steht
sowie Salzen der Verbindungen mit der Formel 111.

4. Verfahren nach einem der Ansprüche 1 bis 3, **dadurch gekennzeichnet, dass** der Katalysator ausgewählt ist aus Aminen mit der allgemeinen Formel IV
NHR¹⁴R¹⁵ (IV)
in der
R¹⁴ für H, eine Kohlenwasserstoffgruppe, wie eine gesättigte oder ungesättigte, verzweigte oder lineare Alkylgruppe, Alkenylgruppe, Alkinylgruppe, Aryl, Alkylaryl, die geeignete Substituenten haben kann, eine Heteroatom-enthaltende Kohlenwasserstoffgruppe, die geeignete Substituenten haben kann, steht, und
R¹⁵ für eine Kohlenwasserstoffgruppe, wie eine gesättigte oder ungesättigte, verzweigte oder lineare Alkylgruppe, Alkenylgruppe, Alkinylgruppe, Aryl, Alkylaryl, die geeignete Substituenten haben kann, eine Heteroatom-enthaltende Kohlenwasserstoffgruppe, die geeignete Substituenten haben kann, steht,
wobei R¹⁴ und R¹⁵ einen anellierten, substituierten oder unsubstituierten Ring mit 3 bis 7 Kohlenstoffatomen bilden können, der zusätzlich zum N-Atom aus Formel IV ggf. eine weiteres Heteroatom enthalten kann,
und deren Säureadditionssalze.

5. Verfahren nach Anspruch 4, **dadurch gekennzeichnet, dass** das Amin ausgewählt ist aus Imidazolidinon und dessen Derivate mit der allgemeinen Formel V 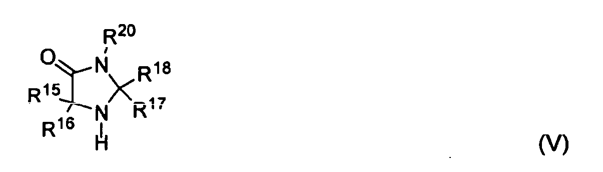 in der
R¹⁵, R¹⁶, R¹⁷ und R¹⁸ gleich oder verschieden sein können und für H, OH, F, Cl, Br, J, NO₂, NO, SO₂, SO₃⁻, Amino, Mono- und Di-(C₁-C₂₄-alkyl)-substituiertes Amino, Mono-und Di-(C₅-C₂₀-aryl)-substituiertes Amino, Imino, Phosphono, Phosphonato, Phosphinato, Phospho, Phosphino, einen verzweigten oder unverzweigten, gesättigten oder ungesättigten Kohlenwasserstoffrest mit 1 bis 30 Kohlenstoffatomen, der geeignete Substituenten haben kann, wobei der Kohlenwasserstoffrest ein oder mehrere Heteroatome in der Kette aufweisen kann, eine Arylgruppe oder Heteroarylgruppe, die geeignete Substituenten haben können, stehen, wobei die Reste R¹⁷ und/oder R¹⁸ mit R²⁰ einen 5- oder 6-gliedrigen Ring bilden können, der aromatisch, alicyclisch, heteroaromatisch oder heteroalicyclisch sein kann und bis zu 4 Substituenten aufweisen kann,
R²⁰ für H, eine Kohlenwasserstoffgruppe, wie eine gesättigte oder ungesättigte, verzweigte oder lineare Alkylgruppe, Alkenylgruppe, Alkinylgruppe, Aryl, Alkylaryl, die geeignete Substituenten haben kann, eine Heteroatom-enthaltende Kohlenwasserstoffgruppe, die geeignete Substituenten haben kann, steht.
Der verwendete Begriff "Alkyl" bedeutet einen linearen, verzweigten oder cyclischen Kohlenwasserstoffrest.
Der verwendete Begriff "Alkenyl" bedeutet einen linearen, verzweigten oder cyclischen Kohlenwasserstoffrest mit mindestens einer Doppelbindung,
Der verwendete Begriff "Alkinyl" bedeutet einen linearen, verzweigten oder cyclischen Kohlenwasserstoffrest mit mindestens einer Dreifachbindung,

## Claims

1. Process for hydrogenating α,β-unsaturated carbonyl compounds, in which a compound with the general formula I 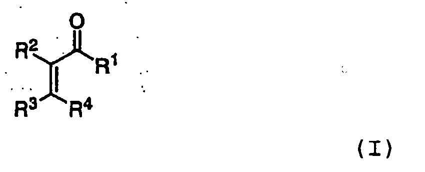 in which
R¹ is H, a branched or unbranched, saturated or unsaturated hydrocarbon radical which has from 1 to 30 carbon atoms and may have suitable substituents, where the hydrocarbon radical may have one or more heteroatoms in the chain, an aryl group or heteroaryl group which may have suitable substituents,
R² , R³ and R⁴ may be the same or different and are H, F, Cl, Br, I, OH, CN, NO₂, NO, SO₂, SO₃⁻, amino, mono- and di-(C₁-C₂₄-alkyl)-substituted amino, mono- and di-(C₅-C₂₀-aryl)-substituted amino, imino, phosphono, phosphonato, phosphinato, phospho, phosphino, a branched or unbranched, saturated or unsaturated hydrocarbon radical which has from 1 to 30 carbon atoms and may have suitable substituents, where the hydrocarbon radical may have a heteroatom in the chain, an aryl group or heteroaryl group which may have suitable substituents,
where in each case 2 or more of the R¹, R², R³ and R⁴ radicals may form a 5- or 6-membered ring or fused 5-membered and/or 6-membered rings, which may be aromatic, alicyclic, heteroaromatic or heteroalicyclic and may have up to 4 substituents, is reacted with a hydride donor to form a compound with the general formula II 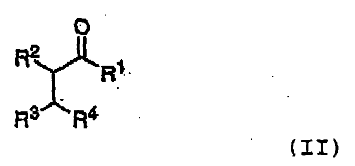
in which R¹, R², R³ and R⁴ are each as defined above, **characterized in that** the redaction is performed in the presence of a catalyst which its selected from organic bases, especially amines and their acid addition salts. The term "alkyl" used means a linear, branched or cyclic hydrocarbon radicals.

2. Process according to Claim 1, **characterized in that** the hydride donor is selected from dihydropyridines, secondary alcohols, silanes, triarylmethanes, cyclohexadienes, formaldehyde and its derivatives, formic acid and its derivatives and salts.

3. Process according to Claim 2, **characterized in that** the hydride donor used is a dihydropyridine of the general formula III 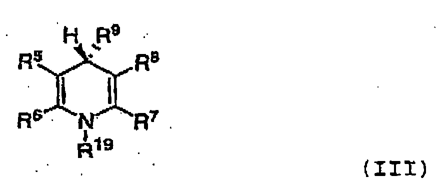 in which R⁵, R⁶, R⁷, R⁸ and R⁹ may be the same or different and are H, OH, a saturated or unsaturated, straight-chain, branched or cyclic, unsubstituted or substituted C₁-C₂₀-alkyl radical, halogen, especially F, Cl, Br, I, NO₂, an amino group, -CO₂R¹⁰, C(O)R¹¹, C-O-R¹², OR¹³, where R¹⁰ to R¹³ may each be selected from H, branched or unbranched, saturated or unsaturated hydrocarbon radicals which have 1 to 30 carbon atoms and may have suitable substituents, where the hydrocarbon radicals may have one or more heteroatoms in the chain, aryl groups or heteroaryl groups, which may in turn have suitable substituents, and
R¹⁹ is H, a branched or unbranched, saturated or unsaturated hydrocarbon radical which has from 1 to 30 carbon atoms and may contain suitable substituents, where the hydrocarbon radicals may have one or more heteroatoms in the chain and suitable substituents, aryl groups or heteroaryl groups which may in turn have suitable substituents,
and salts of the compounds with the formula III.

4. Process according to one of Claims 1 to 3, **characterized in that** the catalyst is selected from amines with the general formula IV
NHR¹⁴R¹⁵ (IV)
in which
R¹⁴ is H, a hydrocarbon group such as a saturated or unsaturated, branched or linear alkyl group, alkenyl group, alkynyl group, aryl, alkylaryl, which may have suitable substituents, or a heteroatom-containing hydrocarbon group which may have suitable substituents, and
R¹⁵ is a hydrocarbon group such as a saturated or unsaturated, branched or linear alkyl group, alkenyl group, alkynyl group, aryl, alkylaryl, which may have suitable substituents, a heteroatom-containing hydrocarbon group which may have suitable substituents,
where Rⁱ⁴ and R¹⁵ may form a fused, substituted or unsubstituted ring which has from 3 to 7 carbon atoms and may, in addition to the nitrogen atom from formula IV, also contain a further heteroatom,
and their acid addition salts.

5. Process according to Claim 4, **characterized in that** the amine is selected from imidazolidinone and its derivatives with the general formula V 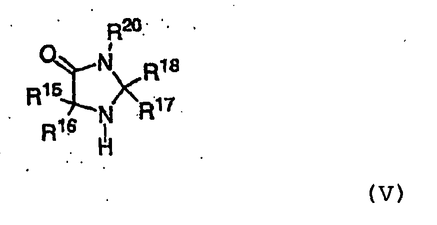 in which
R¹⁵, R¹⁶, R¹⁷ and R¹⁸ may be the same or different and are each H, OH, F, Cl, Br, I, NO₂, NO, SO₂, SO₃⁻, amino, mono- and di-(C₁-C₂₄-alkyl)-substituted amino, mono- and di-(C₅-C₂₀-aryl)-substituted amino, imino, phosphono, phosphonato, phosphinato, phospho, phosphino, a branched or unbranched, saturated or unsaturated hydrocarbon radical which has from 1 to 30 carbon atoms and may have suitable substituents, where the hydrocarbon radical may have one or more heteroatoms in the chain, an aryl group or heteroaryl group which may have suitable substituents, where the R¹⁷ and/or R¹⁸ radicals with R²⁰ may form a 5- or 6-membered ring which may be aromatic, alicyclic, heteroaromatic or heteroalicyclic and may have up to 4 substituents,
R²⁰ is H, a hydrocarbon group such as a saturated or unsaturated, branched or linear alkyl group, alkenyl group, alkynyl group, aryl, alkylaryl, which may have suitable substituents, a heteroatom-containing hydrocarbon group which may have suitable substituents.
The term "alkyl" used means a linear, branched or cyclic hydrocarbon radical. The term "alkenyl" used means a linear, branched or cyclic hydrocarbon radical with at least one double bond. The term "alkynyl" used means a linear, branched or cyclic hydrocarbon radical with at least one triple bond.

## Revendications

1. Procédé d'hydrogénation de composés carbonyle α,β-insaturés, dans lequel on transforme un composé présentant la formule générale I 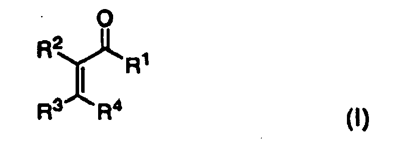 dans laquelle
R¹ représente H, un radical hydrocarboné ramifié ou non ramifié, saturé ou insaturé, comprenant 1 à 30 atomes de carbone, qui peut présenter des substituants appropriés, où le radical hydrocarboné peut présenter un ou plusieurs hétéroatomes dans la chaîne, un groupe aryle ou un groupe hétéroaryle, qui peuvent présenter des substituants appropriés,
R² R³ et R⁴ peuvent être identiques ou différentes et représentent H, F, Cl, Br, I, OH, CN, NO₂, NO, SO₂, SO₃⁻, amino, amino substitué par mono-(C₁-C₂₄- alkyle) et di-(C₁-C₂₄-alkyle), amino substitué par mono-(C₅-C₂₀-aryle) et di-(C₅-C₂₀-aryle) imino, phosphono, phosphonato, phosphinato, phospho, phosphino, un radical hydrocarboné ramifié ou non ramifié, saturé ou insaturé, comprenant 1 à 30 atomes de carbone, qui peut présenter des substituants appropriés, où le radical hydrocarboné peut présenter un hétéroatome dans la chaîne, un groupe aryle ou un groupe hétéroaryle, qui peuvent présenter des substituants appropriés,
où à chaque fois 2 des radicaux R¹, R², R³ et R⁴ ou plus peuvent former un cycle de 5 ou 6 chaînons ou des cycles annelés de 5 et/ou de 6 chaînons, qui peuvent être aromatiques, alicycliques, hétéroaromatiques ou hétéroalicycliques et qui peuvent présenter jusqu'à 4 substituants,
avec un donneur d'hydrure avec formation d'un composé présentant la formule générale II 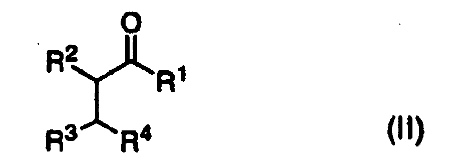 dans laquelle R¹, R², R³ et R⁴ sont définis comme ci-dessus,
**caractérisé en ce que** la transformation est réalisée en présence d'un catalyseur, qui est choisi parmi les bases organiques, en particulier les amines et leurs sels d'addition d'acide, le concept "alkyle" utilisé signifiant un radical hydrocarboné linéaire, ramifié ou cyclique.

2. Procédé selon la revendication 1, **caractérisé en ce que** le donneur d'hydrure est choisi parmi les dihydropyridines, les alcools secondaires, les silanes, les triarylméthanes, les cyclohexadiènes, le formaldéhyde et ses dérivés, l'acide formique et ses dérivés et sels.

3. Procédé selon la revendication 2, **caractérisé en ce qu'**on utilise comme donneur d'hydrure une dihydropyridine de formule générale III 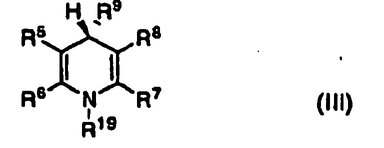 dans laquelle
R⁵, R⁶, R⁷, R⁸ et R⁹ peuvent être identiques ou différents et représentent H, OH, un radïcal C₁- C₂₀-alkyle saturé ou insaturé, linéaire, ramifié ou cyclique, non substitué ou substitué, halogène, en particulier F, Cl, Br, I, NO₂, un groupe amino, -CO₂R¹⁰, C(O)R¹¹ , C-O-R¹², OR¹³, où R¹⁰ à R¹³ peuvent être choisis parmi H, des radicaux hydrocarbonés ramifiés ou non ramifiés, saturés ou insaturés comprenant 1 à 30 atomes de carbone, qui peuvent présenter des substituants appropriés, où les radicaux hydrocarbonés peuvent présenter un ou plusieurs hétéroatomes dans la chaîne, des groupes aryle ou hétéroaryle qui peuvent à leur tour présenter des substituants appropriés, et
R¹⁹ représente H, un radical hydrocarboné ramifié ou non ramifié, saturé ou insaturé, comprenant 1 à 30 atomes de carbone, qui peut présenter des substituants appropriés, où les radicaux hydrocarbonés peuvent présenter un ou plusieurs hétéroatomes dans la chaîne ainsi que des substituants appropriés, des groupes aryle ou hétéroaryle, qui peuvent présenter à leur tour des substituants appropriés,
ainsi que des sels des composés de formule III.

4. Procédé selon l'une quelconque des revendications 1 à 3, **caractérisé en ce que** le catalyseur est choisi parmi les amines de formule générale IV
NHR¹⁴R¹⁵ (IV)
dans laquelle
R¹⁴ représente H, un groupe hydrocarboné, tel qu'un groupe alkyle, un groupe alcényle, un groupe alcynyle saturé ou insaturé, ramifié ou linéaire, aryle, alkylaryle, qui peut présenter des substituants appropriés, un groupe hydrocarboné contenant des hétéroatomes, qui peut présenter des substituants appropriés et
R¹⁵ représente un groupe hydrocarboné, tel qu'un groupe alkyle, un groupe alcényle, un groupe alcynyle saturé ou insaturé, ramifié ou linéaire, aryle, alkylaryle, qui peut présenter des substituants appropriés, un groupe hydrocarboné contenant des hétéroatomes, qui peut présenter des substituants appropriés,
où R¹⁴ et R¹⁵ peuvent former un cycle annelé, substitué ou non substitué comprenant 3 à 7 atomes de carbone, qui peut contenir, en plus de l'atome de N de la formule IV, le cas échéant un autre hétéroatome,
et leurs sels d'addition d'acide.

5. Procédé selon la revendication 4, **caractérisé en ce que** l'amine est choisie parmi l'imidazolidinone et ses dérivés de formule générale V 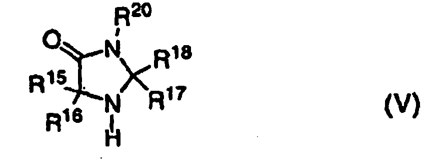 dans laquelle
R¹⁵, R¹⁶, R¹⁷ et R¹⁸ peuvent être identiques ou différents et représentent H, OH, F, Cl, Br, I, NO₂, NO, SO₂, SO₃⁻, amino, amino substitué par mono- (C₁-C₂₄-alkyle) et di-(C₁-C₂₄-alkyle), amino substitué par mono-(C₅-C₂₀-aryle) et di- (C₅-C₂₀- aryle), imino, phosphono, phosphonato, phosphinato, phospho, phosphino, un radical hydrocarboné ramifié ou non ramifié, saturé ou insaturé comprenant 1 à 30 atomes de carbone, qui peut présenter des substituants appropriés, où le radical hydrocarboné peut présenter un ou plusieurs hétéroatomes dans la chaîne, un groupe aryle ou un groupe hétéroaryle, qui peuvent présenter des substituants appropriés, où les radicaux R¹⁷ et/ou R¹⁸ peu (ven) t former avec R²⁰ un cycle de 5 ou 6 chaînons, qui peut être aromatique, alicyclique, hétéroaromatique ou hétéroalicyclique et qui peut présenter jusqu'à 4 substituants,
R²⁰ représente H, un groupe hydrocarboné, tel qu'un groupe alkyle, un groupe alcényle, un groupe alcynyle saturé ou insaturé, ramifié ou linéaire, aryle, alkylaryle, qui peut présenter des substituants appropriés, un groupe hydrocarboné contenant des hétéroatomes, qui peut présenter des substituants appropriés,
le concept "alkyle" utilisé signifie un radical hydrocarboné linéaire, ramifié ou cyclique,
le concept "alcényle" utilisé signifie un radical hydrocarboné linéaire, ramifié ou cyclique, comprenant au moins une double liaison,
le concept "alcynyle" utilisé signifie un radical hydrocarboné linéaire, ramifié ou cyclique, comprenant au moins une triple liaison.
